# EUROPEAN PATENT SPECIFICATION

(11) **EP 1 490 077 B1**
(45) Date of publication and mention of the grant of the patent: **21.11.2007**
(21) Application number: 03742551.9
(22) Date of filing: 18.02.2003
(51) Int. Cl.: A61K 35/74, A61K 36/06, A61K 36/064, A61P 17/16, A61K 8/99, A61Q 17/04

(54) **A PHOTOPROTECTIVE ORALLY ADMINISTRABLE COMPOSITION FOR SKIN**
PHOTOPROTEKTIVE ORAL VERABREICHBARE ZUSAMMENSETZUNG FÜR DIE HAUT
COMPOSITION PHOTOPROTECTRICE POUR LA PEAU A ADMINISTRER ORALEMENT

(30) Priority: 21.02.2002 EP 02075701
(43) Date of publication of application: 29.12.2004
(73) Proprietor: SOCIETE DES PRODUITS NESTLE S.A., 1800 Vevey (CH); L'ORÉAL, 75008 Paris (FR)
(72) Inventor: BRETON, Lionel, F-78000 Versailles (FR); BUREAU-FRANZ, Isabelle, CH-1052 Le Mont-Sur-Lausanne (CH); GUENICHE, Audrey, F-92500 Rueil Malmaison (FR)
(86) International application number: PCT/EP2003/001686
(87) International publication number: WO 2003/070260

(56) References cited:
- EP-A- 0 931 543
- EP-A- 1 222 919
- WO-A-00/33854
- WO-A-96/26732
- FR-A- 2 718 752
- FR-A- 2 725 896
- US-A- 3 920 834
- US-A- 4 464 362
- US-A- 5 397 773
- US-A- 5 501 857
- US-A- 6 110 478
- PATENT ABSTRACTS OF JAPAN vol. 017, no. 291 (C-1067), 4 June 1993 (1993-06-04) & JP 05 017363 A (YAKULT HONSHA CO LTD), 26 January 1993 (1993-01-26)
- DATABASE CA [Online] CHEMICAL ABSTRACTS SERVICE, COLUMBUS, OHIO, US; SASAKI, MINORU ET AL: "Rough skin -preventing cosmetics containing ceramide formation promoters" retrieved from STN Database accession no. 127:195256 XP002209045 & JP 09 194383 A (KANEBO, LTD., JAPAN) 29 July 1997 (1997-07-29)

## Description

### Field of the invention

The present invention relates to the use of a photoprotecting effective amount of i) at least one yeast and ii) at least one strain of probiotic lactic acid bacterium for preparing an orally admistrable composition for the photoprotection of the skin, whether before, during and/or after exposure to UV radiation, and for preventing and/or attenuating the damage caused by such UV irradiation.

### Background of the Invention

The continuous decrease of the atmosphere's ozone layer with the concurrent increase of ultraviolet radiation reaching the planet's surface has attracted a great deal of interest in its potential consequence on human health.

Indeed, it is known that light radiation of wavelengths of from 320 nm to 400 nm (UV-A) promotes tanning of the human epidermis; such radiation, however, is likely to cause damage to the skin, especially in the case of sensitive skin or skin which is continuously exposed to solar radiation. UV-A rays cause, in particular, a loss in the elasticity of the skin and the appearance of wrinkles, promoting a premature ageing thereof. It is also known to this art that light rays having wavelengths of from 280 to 320 nm (UV-B) cause erythema and skin burning which can impair the natural development of a tan.

Although exposure to ultraviolet radiation is needed for humans to produce vitamin D, growing evidence suggests that extensive exposure to sun-light, in particular to ultraviolet radiation, causes a variety of problems in the skin, including induction of certain skin cancers and induction of accelerated skin ageing.

In addition to these established health concerns, research has also provided evidence suggesting that exposure to ultraviolet radiation may negatively affect a variety of immune responses in living beings both locally, within the UV-irradiated skin, and also systemically, i.e. at sites distant from the irradiated skin.

It is thus necessary, in order to maintain suitable skin quality after exposure to UV radiation, to prepare or treat the skin before the exposure, to protect it during the exposure and even to alleviate the detrimental effects of ultraviolet radiation on the skin, prevent the development of erythema, oedema and/or flaking or scaling (hyperkeratosis) of the skin.

In the art, there have been several attempts, such as by using sunscreens or other particular pharmacological agents.

In J. Invest. Dermatol., 97 (1991), 624-628 it is reported that topical application of ultraviolet radiation-absorbing compounds (sunscreens) is effective in preventing ultraviolet radiation-induced erythema and edema but cannot prevent UV-light induced immuno-suppression. This finding was confirmed by several other studies, according to which sunscreens seems to prevent inflammation or irritation but do not provide complete prophylactic protection against the immuno-suppressive effects of ultraviolet radiation.

On the other hand, In FR 2698 268 (L'Oreal) an orally administrable composition comprising a combination of at least one amino-acid, salt of copper and a mix of vitamins has been shown to protect the skin against ultraviolet radiation.

However, there is still a need in the art for an orally administrable composition which is capable to improve and/or reinforce the photoprotective function of the skin.

### Summary of the invention

Accordingly, the present invention aims to provide an orally administrable composition for the photoprotection of the skin which comprises a photoprotecting effective amount of i) at least one probiotic lactic acid bacterium or a culture supernatant thereof, and ii) at least one yeast, included into an orally acceptable carrier.

The present invention relates to the Use of a photoprotecting effective amount of at least one probiotic lactic acid bacterium or a culture supernatant thereof and at least one yeast, included into an orally acceptable carrier, for preparing an orally administrable composition for the protection of the skin against solar radiation and attenuating or preventing all related skin disorders, such as erythema, inflammation, sun burn, barrier function, photoageing, alteration of the immune system, for example.

The combination according to the present invention has a particular beneficial effect on skin protection and colouration of the skin that helps to reduce the effects of solar radiation-related stress on skin.

### Detailed Description of the Invention

Within the following description, "NCC" designates Nestlé Culture Collection (Nestlé Research Center, Vers-chez-les-Blanc, Lausanne, Switzerland). The term "photoprotection" is used to describe attempt to block or reduce the adverse clinical, histological and immunological effects of solar radiation exposure on the skin.

According to the present invention, the subject compositions comprise, as the active agents therefor, combinatory immixture of at least one probiotic lactic acid bacteria or bifidobacteria or a culture supernatant thereof, and at least one yeast.

Indeed, it has now surprisingly and unexpectedly been determined that admixture of these two very specific constituents elicits an enhanced effect or response in respect of the photoprotection of the skin.

Probiotics are non-pathogenic and non-toxigenic organisms that survive passage through the stomach and small intestine. Upon continuous ingestion by the host they eventually may colonize the gut to a substantial extent thus competing with other potentially pathogenic bacteria for nutrients and/or attachment sites on the gastrointestinal wall and reducing their numbers and reducing or preventing infections. Until now a number of different probiotic micro-organisms have been found, which all are reported to exert their effect in the gut via the production of toxins, metabolic by-products, short chain fatty acids and the like.

It has now been shown that probiotics do also exert an effect in an individual's body at a location distant from the region in which they colonize it. And particularly, it has been surprisingly found that a composition having a synergistic photoprotective effect on the skin may be obtained by combining into an orally acceptable carrier, a probiotic microorganism and yeast.

In a preferred embodiment, the probiotic to be included into the carrier is selected from the group consisting of lactic acid bacteria, in particular Lactobacilli and/or Bifidobacteria and are more preferably *Lactobacillus johnsonii, Lactobacillus reuteri, Lactobacillus rhamnosus, Lactobacillus paracasei, Lactobacillus casei* or *Bifidobacterium bifidum, Bifidobacterium breve, Bifidobacterium longum, Bifidobacterium animalis*, Bifidobacterium lactis, *Bifidobacterium infantis*, *Bifidobacterium adolescentis* or *Bifidobacterium pseudocatenulatum*, or a mixture thereof.

According to a most preferred embodiment the strains *Lactobacillus johnsonii* NCC 533, *Lactobacillus paracasei* NCC 2461, *Bifidobacterium adolescentis* NCC 251 and *Bifidobacterium longum* NCC 490 were deposited by way of an example, under the Budapest Treaty with the Institut Pasteur (28 rue du Docteur Roux, F-75024 Paris cédex 15) on 30.06.92, 12.01.99, 15.04.99 and 15.03.99, respectively and under the deposit number CNCM I-1225, CNCM I-2116, CNCM I-2168 and CNCM I-2170, respectively.

The strain of *Bifidobacterium lactis* (ATCC27536) provided by Hansen (Chr. Hansen A/S, 10-12 Boege Alle, P.O. Box 407, DK-2970 Hoersholm, Danemark) can also be used.

The probiotic microorganism according to the present invention may be included in a live form, semi-active or in deactivated form, e.g. as a lyophilized powder. Also culture supernatants of the microorganisms may be included in the products, optionally in concentrated form. It may also be included in an encapsulated form. When using a supernatant of a probiotic's culture the supernatant may be used as such or may be subjected to one or more purification steps prior to inclusion into the product, so as to concentrate or isolate the active ingredient (s) /metabolite (s). Method and techniques for purifying compounds and detecting the activity thereof in the fractions obtained are well known to the skilled person.

The probiotic lactic acid bacteria may be present in the carrier in an amount of at least 10⁵ cfu/ g of orally acceptable carrier, preferably from about 10⁵ to 10¹⁵ cfu/ g of orally acceptable carrier, and more preferably from 10⁷ to 10¹²cfu/ g of orally acceptable carrier.

It may be incorporated in dispersion form in a suitable vehicle such as water, organic solvents and fatty substances including oils, whether alone or in admixture.

The compositions according to the invention also comprise yeast. In a preferred embodiment, the yeast is any food-grade yeast selected from the group consisting of Ascomycotina or Deuteromycotina . In a preferred embodiment, the yeast may be selected from the group consisting of *Debaryomyces, Kluyveromyces, Saccharomyces, Yarrowia, Zygosaccharomyces, Candida and Rhodutorula*, and more preferably *Saccharomyces caerevisae* (baker's yeast).

Such yeast may be used in the form of dried or lyophilized extracts. It may be present in the carrier in an amount of at least 10⁵ cfu/ g of orally acceptable carrier, preferably from about 10⁵ to 10¹⁵ cfu/ g of orally acceptable carrier, and more preferably from 10⁷ to 10¹²cfu/ g of orally acceptable carrier, said amount depending on the nature and activity of the particular yeast.

A mixture of a plurality of lactic acid bacteria or yeast may also be used.

The composition may also comprise a third photoprotecting agent, preferably at least one carotenoid with or without provitamin A activity, such as β-carotene, γ-carotene, α-carotene, lycopene, zeaxanthine and luteine, or a mixture thereof. The carotenoid may be from synthetic or natural origin or contained in a natural extract. When the carotenoid is from natural origin, it is preferably obtained from plant material, in which the plant is grown in-vivo or in-vitro. Method for extracting the carotenoids is well known in the art. The carotenoid may be present in the carrier in an amount of from 10⁻¹²% to 20% by weight and preferably from 0,00001 mg to 50 mg/day and more preferably from 0.001mg to 30mg/day.

The carrier may be any food or pharmaceutical product, or a nutritional supplement or a composition for oral administration, wherein the probiotic microorganism and the yeast may be included. Examples for food or pharmaceuticals carriers are milk, yoghurt, curd, cheese, fermented milks, milk based fermented products, ice-creams, fermented cereal based products, milk based powders, infant formulae or tablets, liquid suspensions, dried oral supplement, wet oral supplement, dry-tube-feeding. The composition for oral administration may be in capsules, soft capsules, tablets, pastes or pastilles, gums, or drinkable solutions or emulsions. Methods for preparing the carrier are common knowledge.

The composition according to the invention may also comprise usual excipients, in particular sweeteners, flavouring agents or preservatives.

The compositions of the invention may be formulated according to any one of a number of techniques that are well known to this art.

The composition according to the invention provides a surprising and synergistic protective and preventive effect of the skin.

The amount of the composition to be consumed by the individual will depend on the desirable effect. However, an amount of the composition to provide a daily amount of about 10⁵ to 10¹² organisms, which organism may be alive or dead, would usually be adequate.

The composition is administered to an individual before or during the exposure to ultraviolet radiations, in particular exposure to sun. When the exposure period is foreseeable, it is desirable to start the consumption before the exposure and preferably 1 to 2 months before, and to prolong consumption during exposure.

In order to further illustrate the present invention and the advantages thereof, the following specific examples are given, it being understood that same are intended only as illustrative and in nowise limitative.In said examples to follow, as in the above description, all parts and percentages are given by weight, unless otherwise indicated.

### Examples

In the following examples 1 to 6, β-carotene is provided by Roche, Lycopene is provided by Lycored, Lyophilized *S.cerevissae* is provided by BioSpringer, *Latobacillus* CNCM I-1225 dry mix, *Lactobacillus* CNCM I-2116 or *Bifidobacterium* CNCM I-2168 dry mix are prepared so that they contain 1.10⁸ to 1.10⁹ organisms.

### Example 1 :

A photoprotective daily orally administrable composition is prepared as follows:

| | |
|---|---|
| Lyophilized *S.cerevissae* | 75 mg |
| Latobacillus CNCM I-1225 dry mix | 50 mg |
| Glucidex IT 19 (maltodextrin powder) | QSP 500 mg |

The composition is administered to the individual in an amount of 2x500 mg daily, which provides a protective and preventive effect of the skin.

### Example 2 :

A photoprotective daily orally administrable composition is prepared as follows:

| | |
|---|---|
| Lyophilized *S.cerevissae* | 75 mg |
| Bifidobacterium CNCM I-2168 dry mix | 50 mg |
| β-carotene | 4.7 mg |
| Glucidex IT 19 (maltodextrin powder) | QSP 500 mg |

The composition is administered to the individual in an amount of 2x500 mg daily, which provides a protective and preventive effect of the skin.

### Example 3 :

A photoprotective daily orally administrable composition is prepared as follows:

| | |
|---|---|
| Lyophilized *S.cerevissae* | 75 mg |
| Latobacillus CNCM I-1225 dry mix | 50 mg |
| β-carotene | 4.7 mg |
| Lycopene | 2.5 mg |
| Glucidex IT 19 (maltodextrin powder) | QSP 500 mg |

The composition is administered to the individual in an amount of 2x500 mg daily, which provides a protective and preventive effect of the skin.

### Example 4:

A photoprotective daily orally administrable composition is prepared as follows:

| | |
|---|---|
| Lyophilized *S.cerevissae* | 75 mg |
| Latobacillus CNCM I-2116 dry mix | 50 mg |
| β-carotene | 4.7 mg |
| Zeaxanthine | 10 mg |
| Glucidex IT 19 (maltodextrin powder) | QSP 500 mg |

The composition is administered to the individual in an amount of 2x500 mg daily, which provides a protective and preventive effect of the skin.

### Example 5 :

A photoprotective daily orally administrable composition is prepared as follows:

| | |
|---|---|
| Lyophilized *S.cerevissae* | 75 mg |
| Bifidobacterium CNCM I-2168 | 30 mg |
| Latobacillus CNCM I-1225 dry mix | 30 mg |
| β-carotene | 4.7 mg |
| Lycopene | 2.5 mg |
| Glucidex IT 19 (maltodextrin powder) | QSP 500 mg |

The composition is administered to the individual in an amount of 2x500 mg daily, which provides a protective and preventive effect of the skin.

## Claims

1. Use of a photoprotecting effective amount of at least one probiotic lactic acid bacterium or a culture supernatant thereof and at least one yeast, included into an orally acceptable carrier, for preparing an orally administrable composition for the protection of the skin against solar radiations and attenuating or preventing all related skin disorders.

2. The use according to claim 1, in which the lactic acid bacterium is *Lactobacillus johnsonii, Lactobacillus reuteri, Lactobacillus rhamnosus, Lactobacillus paracasei, Lactobacillus casei* or *Bifidobacterium bifidum, Bifidobacterium breve, Bifidobacterium longum, Bifidobacerium animalis, Bifidobacterium lactis, Bifidobacterium infantis, Bifidobacterium adolescentis, Bifidobacterium pseudocatenulatum*, or a mixture thereof.

3. The use according to claim 1 or 2, wherein the lactic acid bacterium is CNCM I-1225, CNCM I-2116, CNCM I-2168, CNCM I-2170 or ATCC 27536.

4. The use according to one of claims 1 to 3, wherein the yeast is selected from the group consisting of *Debaryomyces, Kluyveromyces, Saccharomyces, Yarrowia Zygosaccharomyces, Candida and Rhodutorula*, or a mixture thereof.

5. The use according to one of claims 1 to 4, wherein the probiotic lactic acid bacterium is present in the carrier in an amount of from about 10⁵ to 10¹⁵ cfu/ g of carrier.

6. The use according to one of claims 1 to 5, wherein the yeast is present in the carrier in an amount of from 10⁵ to 10¹⁵ cfu/ g of carrier.

## Patentansprüche

1. Verwendung einer für den Lichtschutz wirksamen Menge von wenigstens einem probiotischen Milchsäurebakterium oder einem Kulturüberstand davon sowie von wenigstens einer Hefe, die in einen oral annehmbaren Träger eingearbeitet sind, zur Herstellung einer oral verabreichbaren Zusammensetzung zum Schutz der Haut gegen Sonnenstrahlung und zur Abschwächung oder Verhinderung aller damit verbundenen Gesundheitsstörungen der Haut.

2. Verwendung nach Anspruch 1, wobei das Milchsäurebakterium *Lactobacillus johnsonii, Lactobacillus reuteri, Lactobacillus rhamnosus, Lactobacillus paracasei, Lactobacillus casei* oder *Bifidobacterium bifidum, Bifidobacterium breve, Bifidobacterium longum, Bifidobacterium animalis, bifidobacterium lactis, Bifidobacterium infantis, Bifidobacertium adolescentis, Bifidobacterium pseudocatenulatum*, oder eine Mschung davon ist.

3. Verwendung nach Anspruch 1 oder 2, wobei das Milchsäurebakterium CNCM I-1225, CNCM I-2116, CNCM I-2168, CNCM I-2170 oder ATCC 27536 ist.

4. Verwendung nach einem der Ansprüche 1 bis 3, wobei die Hefe ausgewählt ist aus der Gruppe, die besteht aus *Debaryomyces, Kluyveromyces, Saccharomyces, Yarrowia, Zygosaccharomyces, Candida und Rhodutorula*, oder einer Mischung davon.

5. Verwendung nach einem der Ansprüche 1 bis 4, wobei das probiotische Milchsäurebakterium in dem Träger in einer Menge von etwa 10⁵ bis 10¹⁵ KBE/g des Trägers vorhanden ist.

6. Verwendung nach einem der Ansprüche 1 bis 5, wobei die Hefe in dem Träger in einer Menge von 10⁵ bis 10¹⁵ KBE/g des Trägers vorhanden ist.

## Revendications

1. Utilisation d'une quantité photoprotectrice efficace d'au moins une bactérie lactique probiotique ou d'un surnageant de culture de celle-ci et d'au moins une levure, incorporée à un support acceptable par voie orale, pour la préparation d'une composition pouvant être administrée par voie orale, destinée à la protection de la peau contre les rayonnements solaires et à l'atténuation ou la prévention de tous les troubles cutanés correspondants.

2. Utilisation suivant la revendication 1, dans laquelle la bactérie lactique est *Lactobacillus johnsonii, Lactobacillus reuteri, Lactobacillus rhamnosus, Lactobacillus paracasei, Lactobacillus casei* ou *Bifidobacterium bifidum, Bifidobacterium breve, Bifidobacterium longum, Bifidobacterium animalis, Bifidobacterium lactis, Bifidobacterium infantis, Bifidobacterium adolescentis, Bifidobacterium pseudocatenulatum* ou un de leurs mélanges.

3. Utilisation suivant la revendication 1 ou 2, dans laquelle la bactérie lactique est CNCM 1-1225, CNCM 1-2116, CNCM I-2168, CNCM 1-2170 ou ATCC 27536.

4. Utilisation suivant une des revendications 1 à 3, dans laquelle la levure est choisie dans le groupe consistant en *Debaryomyces, Kluyveromyces, Saccharomyces, Yarrowia, Zygosaccharomyces, Candida* et *Rhodutorula* ou un de leurs mélanges.

5. Utilisation suivant une des revendications 1 à 4, dans laquelle la bactérie lactique probiotique est présente dans le support en une quantité d'environ 10⁵ à 10¹⁵ ufc/g de support.

6. Utilisation suivant une des revendications 1 à 5, dans laquelle la levure est présente dans le support en une quantité de 10⁵ à 10¹⁵ ufc/g de support.
